# EUROPEAN PATENT SPECIFICATION

(11) **EP 1 857 079 B1**
(45) Date of publication and mention of the grant of the patent: **05.08.2009**
(21) Application number: 04766933.8
(22) Date of filing: 08.09.2004
(51) Int. Cl.: A61F 2/44

(54) **UNIVERSAL INTERVERTEBRAL DISC PROSTHESIS**
UNIVERSAL-BANDSCHEIBENPROTHESE
PROTHESE DE DISQUE INTERVERTEBRAL A APPLICATION UNIVERSELLE

(43) Date of publication of application: 21.11.2007
(73) Proprietor: Synthes GmbH, 4436 Oberdorf (CH)
(72) Inventor: GARCÍA VACAS, Francisco Manuel, E-29009 Málaga (ES); EZQUERRO JUANCO, Francisco, E-29631 Málaga (ES); SIMÓN MATA, Antonio, E-29012 Málaga (ES)
(74) Representative: Rosenich, Paul
(86) International application number: PCT/ES2004/000395
(87) International publication number: WO 2006/042870

(56) References cited:
- WO-A1-01/01893
- WO-A2-02/11650
- ES-T3- 2 027 759
- ES-T3- 2 057 874
- ES-T3- 2 094 393
- ES-T3- 2 163 216
- FR-A1- 2 824 261
- FR-A1- 2 846 550
- US-A- 4 759 766
- US-A- 5 893 889
- US-A1- 2004 034 424
- US-A1- 2004 054 411

## Description

### 1. OBJECT OF THE INVENTION

The object of this invention is to provide a new model of Modular Disc Prosthesis designed to reproduce the functions of the natural human intervertebral disc by providing controlled, total physiological mobility without overloading the small posterior joints, with the possibility of adapting the implant to each particular case according to the degree of disc degeneration which each patient experiences at the time. To achieve these two objectives a prosthesis has been developed: a modular prosthesis which can be used in three versions, constrained, semi-constrained and unconstrained, so that it can be adapted to each patient; with an anatomical-functional design which enables it to be implanted and gives it a solid and firm primary stability due to its covering and the form of anchorage to the bone. This implant is of the low friction type due to the materials used on its sliding surfaces, these materials being high wear resistance. It can be implanted from the front, as all current implants, and also laterally.

The object of this invention is achieved with a disc prosthesis as claimed in the attached claims.

### 2. BACKGROUND

To date there have been many attempts to develop a substitute for the human intervertebral disc, which may degenerate due to premature wear, arthrosis and repeated traumas. The first possible solution for solving this problem, widely used later, was the total replacement of the disc as a mobile element by bone grafts, which give rise to total fusion or welding and the connection of the two contiguous vertebrae, supplied by vertebral plate and screw fixing systems (so-called vertebral fixers), which are inserted via the pedicles of the vertebrae. However, the problem was not finally solved by arthrodesis or fusion, i.e. by eliminating the mobility of a vertebral segment formed by two contiguous vertebrae interconnected by an intervertebral disc, particularly in young patients. When one or more consecutive discs are removed in these patients, the initial problem is solved temporarily, but all the work is transferred to the immediately superior or inferior free disc, as well as the biomechanical stresses corresponding to the fused disc or discs. In this case the supra- or infrajacent discs start to degenerate gradually after a few years until they are totally destroyed, generating what is known as Adjacent Disc Syndrome or Transition Space Syndrome.

To avoid the necessity of having to resolve this Primary Degenerative Disc Pathology by a method that is as restrictive as fusion, and which may later generate a new ascending or descending Progressive Degenerative Pathology, it was decided to develop the Disc Prostheses.

Within this Degenerative Intervertebral Disc Pathology we may find two types of degenerated discs which display totally different biomechanical behaviours:
a) Hypermobile Degenerated Disc peculiar to younger persons, particularly middle aged women, and **characterised in that** it has a range of movement in the three axes of the space at a much higher level than normal, transferring the physiological limits permitted mainly in flexo-extension and in the axial rotations, which normally gives rise to a symptomatology of painful lumbalgias and intermittent pseudo-sciaticas. This situation generally corresponds to the first stages of the Vertebral Instability Syndrome of Kirkaldi and Wyllis.
b) Hypomobile Degenerated Disc, where the mobility of the segment is greatly reduced, below the physiological requirements, occurring mainly in elderly persons and generally corresponding to more advanced stages of Vertebral Instability Syndrome. In these cases the intervertebral space is very small and calcifications, osteophyses and restorative and degenerative phenomena occur in the small joints, with hypertrophies of the same and rigidity of all the elements elastically retained, such as the ligaments and articular capsules of the segment.

Among the two types of discs, hyper- and hypomobile, can be found cases of Normo-Mobile Degenerative Discopathies, where the range of mobility is within normal limits, and the treatment to be given must be different in each case.

All the above must be given particular consideration when selecting the type of prosthesis to be used, which always depends on the type of Degenerative Discopathy suffered by the patient. Each individual case must be studied very exhaustively, both clinically and radiologically, with all kinds of imaging studies (dynamic X-rays, computerised axial tomography, magnetic resonance, etc.) so as to establish what type of Degenerative Discopathy the patient presents and thereby select the type of prosthesis required.

### 3. STATE OF THE ART

Currently over 127 different models of Disc Prostheses are registered, almost 98% of which have never been used in a human being. Of those that have been used, many have broken, due to a design fault, lack of stability or because they are made from unsuitable materials. However, there are some models now on the market which have been used in a large number of patients and which have been applied for over 17 years and are fully accepted by international organisations for controlling implants and prostheses in the human body.

Although it is true that the latest designs have generally improved in terms of their morphology and quality of materials, it is no less certain that they suffer from major defects which justify the need to even the configuration of these disc prostheses even more to ensure that they conform more to what the actual function and anatomy of the human intervertebral disc require.

The first implantation of an artificial disc was carried out by Fernström in 1964 (Steel ball bearings). This first disc consisted of a metal ball bearing implanted between two vertebral bodies, and its use was rejected because of the tendency of the implant to sink into the vertebral bodies, coupled with problems of hypermobility of the vertebral segment. From these first implants until the present day numerous models and prototypes of intervertebral disc prostheses have been constructed, many of these designs have been patented and some have been used in surgery.

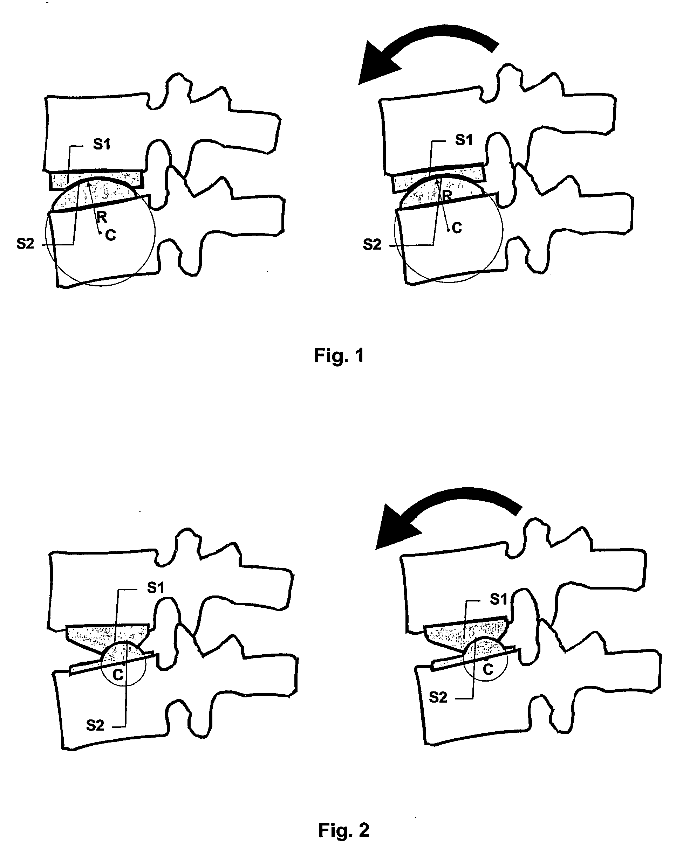

All these designs follow the same main trends: a) they simulate the elastic or visco-elastic behaviour of the disc, in which the energy dissipation capacity or absorption of axial loads predominates; or b) they simulate the characteristics of the natural physiological mobility of the disc.
a) The prostheses which are intended to reproduce the elastic or visco-elastic characteristics of the disc are **characterised in that** they connect both vertebral bodies by means of mechanical elements with suitable elastic properties so that the rigidity they confer upon the whole in the different axes of movement is similar to that corresponding to a true intervertebral disc. In some cases these designs add elements with visco-elastic properties for the purpose of also simulating the visco-elastic properties of the true disc. The materials used to achieve these objectives are mainly silicones, polymers, rubbers or even metal springs. There are various published or patented designs of this type of prosthesis, although very few have actually been implanted.
   These prostheses include: the prosthesis designed by Fassio, rejected due to problems of sinking of the implant; the Acroflex which, after the development of the original design, also appears to have been rejected due to the appearance of defects in the material used, polyolefin, 1-2 years after its installation; and finally the cervical prosthesis from Bryan. None of them has yet been used in practice because the most important thing is that they must reproduce the physiological movement of the disc.
b) For their part, the prostheses designed with the criterion of simulating the mobility characteristics of the intervertebral disc are those most used currently in the surgical treatment of discopathologies because of their improved clinical behaviour. The prostheses emulate the concepts used satisfactorily in the design of prostheses for other joints such as the knee and hip. Their basic function consists in allowing the relative movement between the vertebral bodies that connect by sliding between the surfaces of their different components. Within this type of prosthesis two design trends may be distinguished: firstly those referred to as constrained, and secondly those referred to as unconstrained.
   b-1) The constrained prostheses, which currently include the so-called Pro-Discs, Maverick, Prestige (Bristol) or Flexi-Core, allow the relative rotation between the vertebrae, but do so in such a manner that the axis in respect to which the rotation is performed is determined by the mechanical design of the prosthesis. In these types of prostheses the sliding surfaces are spherical caps, which is why the axis in respect to which the rotations are performed in each anatomical plane is a fixed axis which passes through the geometrical centre of the spheres. The location of each axis is determined by the position of the spherical surfaces within the intervertebral space, as well as by its radius, so that the larger this radius, the further will be the axis of rotation from the intervertebral space. The position of the axis of rotation in turn affects the degree of transversal displacement associated with the rotation, so that generally the lower its position the larger will be the size of the same. (See Fig. 1).
      Among the existing constrained designs may be found a type of design solution based on using sliding surfaces (S1, S2) with a large radius (R) located centrally within the intervertebral space (Fig. 1). In this configuration the centre of rotation (C), both for the flex-extension movements and for the lateral flexion movements, will be located in the central zone of the inferior vertebral body. This position does not coincide with the results of the studies published on the kinematics of the true lumbar column, which place this centre of rotation in different positions along the path of movement, but always around the intervertebral space. Consequently, this type of design will produce a forced movement of the inter-apophysary joints, which would result in an increase in the mechanical stresses supported by the same. his is the case with the Pro-Disc Prosthesis.
      The other solution used in the constrained designs (Maverick and Flexi-Core) is to use sliding surfaces (S1, S2) of a smaller size, which brings the axis of rotation (C) closer to the intervertebral space (Fig. 2), so that a more resistant material must be used in the production of the sliding surfaces. This is because the UHMWPE (Ultra High Molecular Weight Poly Ethylene) used in the previous solutions is unable to resist the high stresses generated in these designs. In the lumbar prostheses the location of the sliding surfaces of these designs tends to be in the posterior third of the intervertebral space. In this location the centre of rotation of the flexo-extension movements can also be found in this posterior zone so that the extension movements of the segment are better simulated but differ from the physiological behaviour in terms of the flexion movements.
      The basic problem from which this types of prostheses suffer is that they do not allow a physiological displacement in the antero-posterior or transversal direction, which constitutes a major defect which imposes a considerable extra load on the small joints, whilst at the same time, since they are provided with only one sliding surface, the degree of friction and wear is far greater and occurs earlier.
      In a true vertebral segment the relative movement between the vertebrae is determined by the interaction between the intervertebral disc, the inter-apophysary joints and the ligaments. The action of each of these structures determines the location of the axis of rotation of this movement, which is variable along the same. Therefore none of the above constrained designs enables the physiological movement of the segment of movement to be restored.
   b-2) The unconstrained designs allow both displacements and independent rotations in the three anatomical axes. This type of design ensures that the axis in respect to which the rotations are produced in each of the anatomical planes is not determined by the prosthesis in itself, but it is the joint action of the prosthesis, ligaments, posterior joints and muscles that determines the position of the said axis in each case.
      The existing unconstrained designs currently include prostheses such as those from Charité and Moby-Disc. Both designs have two pairs of sliding surfaces for transmission of the movement from one vertebra to the other. in the case of Charité, each of the two pairs of surfaces are spheres, whilst in the Moby-Disc prosthesis the pair of upper surfaces are spheres and the lower surfaces are plane.
      Charité was developed in the early 1980's and a large number of them were implanted. Numerous studies were published on this prosthesis, consists of three components: a central polyethylene core and two metal parts containing it. The upper and lower surfaces of the core are convex, and each of them is in contact with the corresponding concave surface of each of the metal parts surrounding the core, these parts being anchored to the vertebral bodies. This design allows the flexo-extension and lateral flexion movements of 2 degrees of freedom by means of both mechanisms. The prosthesis does not therefore force the position of the centre of rotation.
      The Moby-Disc conforms to practically the same concept, but both may in turn give rise to excessive shearing sliding movements which, because they cannot be absorbed by the prosthesis itself, affect the posterior joints by generating scoliosis, which can be painful.
      These types of unconstrained designs provide various possibilities of relative movement between vertebrae, which has the advantage of allowing the vertebral segment to perform a more physiological movement. However, this multiplicity of possible movements may also constitute a disadvantage, as can be the existence of excessive translatory movements which give rise to the transmission of high cutting forces between vertebrae which, because they cannot be absorbed by the prosthesis, will have to be transmitted via the posterior joints. These types of problems mean that the decision as to whether to select constrained and unconstrained prosthesis is not a clear one, which is why both types of prostheses currently coexist on the market, leaving the surgeon to take the final decision as to what type of design he should use.

### Further state of the art

US-A-4759766, published July 26, 1988, discloses an intervertebral disc prosthesis, which can be universally used, of the type formed by three pieces: a lower small plate or piece that is fixed on a lower vertebra, with an upper surface in the form of a spherical cap and with a planar lower surface that ends in a planar border, an upper small plate or piece that is fixed on an upper vertebra, with a lower surface in the form of a spherical cap and with a planar upper surface that ends in a planar border, and a biconcave toroidal intermediate piece that articulates on the other two, lower and upper, pieces by means of individual, upper and lower, cavities in this intermediate piece, which are spherical and have substantially the same curvature as said upper and lower surfaces of the lower and upper pieces, respectively , with the aim that they are in contact over their entire surface when said pieces are mounted in their operative position, such that the top of the spherical surface of the lower piece has a radially oriented pivot pin, in the lowermost part of the spherical surface of the upper piece there is another radially oriented pivot pin, and the intermediate piece has two central circular orifices whose dimensions are greater than those of said pivot pins, in which, when the three pieces that make up the prosthesis are mounted in their operative position, the pivot pins are housed in the circular holes.

### 4. BRIEF DESCRIPTION OF THE DRAWINGS

For a clearer understanding of this description, and since it forms an integral part of the same, a series of figures is attached below which show, by way of illustration and in a non-exhaustive manner, the following:
Fig. 1 shows a set of two vertebrae with an intervertebral disc in two positions of rotation in which the axis of rotation is located in the central zone of the inferior vertebra.
Fig. 2 shows a set of two vertebrae with an intervertebral disc in two positions of rotation in which the axis of rotation is located in the superior zone of the inferior vertebra.
Fig. 3 shows the intervertebral disc prosthesis of the expanded invention, in perspective and in elevation.
Fig. 4 shows a sagittal section of the prosthesis of the invention in its unconstrained configuration.
Fig. 5 shows a perspective view of the prosthesis of the invention in which the left anterior quarter of the same has been omitted for better visualisation.
Fig. 6 shows a perspective view and a plan view of the entirety of the prosthesis of the invention in its configuration for a lateral approach.
Figs. 7a and 7b show sagittal sections of the disc prosthesis of the invention in its constrained and unconstrained configurations respectively.
Figs. 8a and 8b show sagittal sections of the disc prosthesis of the invention, which illustrate the maximum displacements permitted by the said prosthesis in the anterior and posterior directions respectively.
Fig. 9 illustrates an equivalent mechanism of the constrained configuration of the disc prosthesis of the invention.
Fig. 10 shows the instantaneous axes of rotation of the prosthesis of the invention in the flexo-extension movements, position A being the neutral position, B a flexion of 12° relative to A and D being an extension of 9° relative to A.
Fig. 11 shows the differences in the antero-posterior displacement relative to the facet joint when a pair of spherical contact surfaces (left) or the prosthesis of the invention in its constrained configuration (right) is/are used for a 6° flexion.
Fig. 12 shows two sagittal sections of the prosthesis of the invention illustrating the positions adopted by the components of the semi-constrained prosthesis in the maximum ranges of flexion and extension respectively.

### 5. DETAILED DESCRIPTION OF THE INVENTION

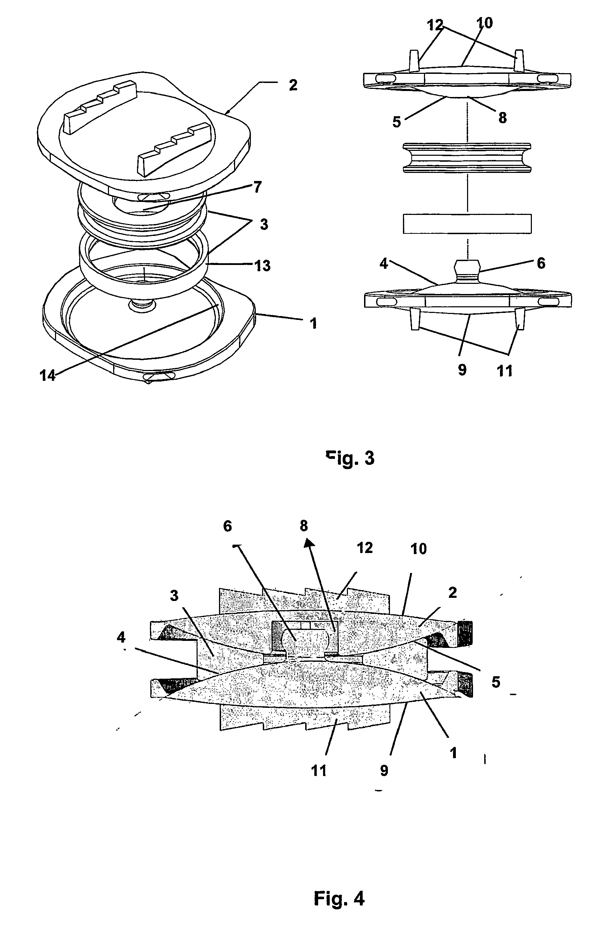

The intervertebral disc prosthesis unit (Figs. 3 and 4) of the invention consists of three different parts:
- a lower part (1) which will be fixed to the inferior vertebra,
- an upper part (2) anchored to the superior vertebra,
both produced in a chromium-cobalt-molybdenum alloy and covered on its outer surface with a tantalum plasma for better osteo-integration.

The lower (1) and upper parts (2) are identical, with one exception that will be indicated below. Both are lenticular in shape in a vertical section and a kidney shape in the plan view. The two parts (1, 2) both have similar spherical surfaces (4, 5, 9, 10) on its upper and lower faces, but the spherical caps forming the lower surfaces (5) of the upper part (2) and upper surface (4) of the lower part (1), which are the operative sliding surfaces in the joint, have a radius of curvature that is smaller than the upper spherical caps (10) of the upper (2) and lower part (9) of the lower part (1). The relative positions of the elements are assumed, according to the prosthesis, in its operative position, as shown in Fig. 4. The operative spherical surfaces (4, 5) terminate on both retaining edges (14), which act as maxima in the displacement of the intermediate part (3). The spherical surfaces (9, 10) opposed to the operative surfaces cover almost all of the upper faces of the upper part (2) and lower face of the lower part (1), which terminate on a flat edge.
- a biconcave toroidal intermediate part (3), manufactured from cross-link ultra high molecular weight polyethylene (CLUHMWPE), which is articulated to the other two parts. The upper and lower cavities of this intermediate part (3) are spherical, with substantially the same curvature as the operative surfaces (4, 5) of the lower (1) and upper parts (2), to ensure that they are in contact, throughout their area, when the said parts (1, 2, 3) are assembled in their operative position. This third polyethylene part is provided with a metal ring or band (13) of titanium around its external circular edge, which confers upon the same a greater resistance whilst serving to visualise its position by means of X-rays. The articulation between the parts (1) and (3), as well as between the parts (2) and (3), is effected by means of sliding between both pairs of spherical surfaces: (4) and (5).

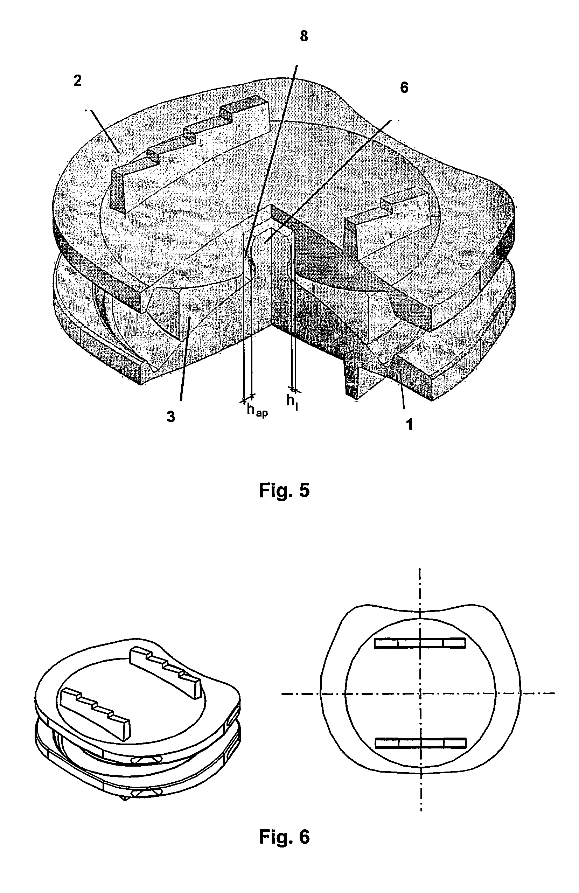

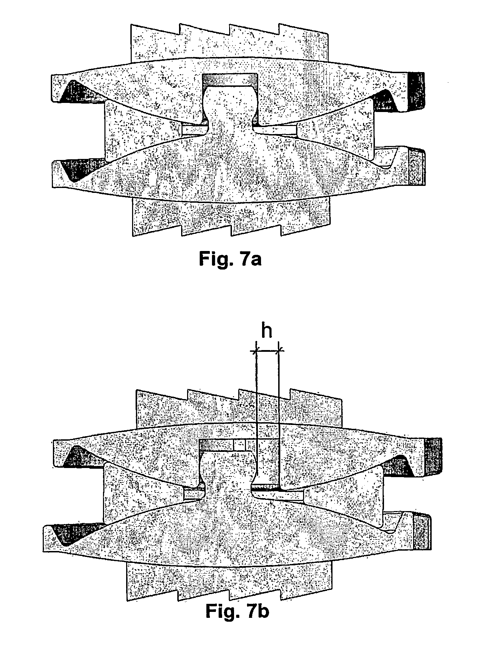

In addition, the lower part (1) has a pivot which departs radially from the top of the surface (4) and ends in a sphere (6). This pivot crosses the intermediate part (3) via the central opening (7), which retains it, and is housed in the hole (8) made in the upper part (2). alternatively, spherical head pivot (6) and the hole (8) may be displaced in the sagittal plane relative to a centred position so that the range of mobility of the prosthesis in flexion is greater than in extension. The adjustment between the sphere of the pivot (6) and the hole (8) depends on the difference between the transversal dimensions of this hole and the diameter of the sphere. Three situations are provided for in designing the prosthesis of this invention:
a) Constrained prosthesis: the hole (8) is cylindrical, with a diameter substantial equal to that of the sphere of the pivot (6) (Fig. 7 A). In this case there is no clearance between the two components, giving rise to a constrained prosthesis design.
b) Semi-constrained prosthesis: the transversal dimensions of the hole (8) are larger than the diameter of the sphere of the pivot (6), giving rise to a clearance between the two elements. This clearance may be characterised by a value h when the hole (8) is cylindrical, or by means of the values: hₐₚ: clearance in the antero-posterior direction and hⱼ: clearance in the medial-lateral direction (Fig. 5 and 7b); in this case the hole (8) is not cylindrical, except that its cross-section is ellipsoidal. In this design situation the clearances h do not exceed 1.5 mm.
c) Unconstrained prosthesis: in this case the clearance h (or hₐₚ, hⱼ) is between 1.5 and 3 mm, allowing greater displacement.

The parts (1) and (2) to the vertebral plates are fixed by means of the external surface of each of these parts, i.e. the lower surface (9) for the lower part (1) and the upper surface (10) of the upper part (2). These surfaces are slightly convex in shape so that they are moulded better to the surface of the vertebral plates, and each of them has two crests or spikes (11) (12) parallel to each other, which must be fixed in the vertebral plates. The design of this prosthesis allows for two possible orientations for these crests, according to the direction of access selected:
1) Parallel to the antero-posterior direction for implantation from the front (Fig. 5).
2) Parallel to the lateral direction for lateral implantation (Fig. 6).

### 6. FUNDAMENTAL ADVANTAGES OF THE DISC PROSTHESIS OF THIS INVENTION

The most important advantages of the prosthesis of the invention are centred on the following aspects: 1. - Osseous anchorage of the vertebral plates; 2. - Modular character of the prosthesis; 3. - Wear and resistance of the material; 4. - Direction of approach, and 5.- Possibility of selecting a suitable type of implant.

### 6.1. - Osseous anchorage.

It is very important to ensure that the prosthesis is fixed and stable at an early stage. this may guarantee initial stability, at least during the first three months, until total osteo-integration is achieved. Even if the vertebral bed is well prepared, it is essential to adjust the contact of the external surface of the prosthesis with the vertebral plates which, in most cases, normally have a concave morphology that is more or less centred and which may leave the prosthesis with a lack of support, and therefore with weak stability that could give rise to premature mobilisation of the same.

To avoid the problem mentioned, the prosthesis of the invention has an external surface with a discrete convexity which allows firm seating on the natural concavity of the vertebral plates. On the contrary, that concave morphology of the vertebral plate requires regulating milling of the same, which is expressed in a weakening of the osseous structure and seating base, and hence the possibility that the prosthesis may sink into the vertebral plate. Therefore the Disk Prosthesis of the invention is provided with external seating surfaces (9, 10) in both discretely convex vertebral plates.

In addition, however, in order to ensure that their adhesion to the bone is as firm as possible, it is necessary, right from the start, for the external surfaces (9, 10) in contact with both vertebral plates to be covered with a material that is biocompatible and easily osteo-integrated, as is the case with the disc prosthesis of the invention, which is covered with a porous tantalum plasma. Moreover, both external surfaces (9, 10) are provided with two lines of metal crests with spikes (11, 12), which will be solidly fixed to the spongy material of the vertebral bodies during the first few weeks of their implantation.

### 6.2 - Modular character of the prosthesis.

The prosthesis of the invention, which may be called modular or multifunctional, may behave as a constrained, unconstrained or semi-constrained prosthesis as required in each case. It has a double sliding surface (each of surfaces 4 and 5 with the corresponding surfaces of the intermediate part)), with the possibility of displacements in both the limited transversal and antero-posterior horizontal directions, and a third metal-metal articulation between the small sphere of the central pivot (6) and its housing (8) on the upper part (2).

The characteristic of being able to act on the constrained or semi-constrained prosthesis is dependent on the relative dimensions given between the sphere (6) of the lower part (1) and the hole (8) in the upper part (2).

As can be seen in Fig. 7a, if the dimensions of the said sphere and the said hole substantially coincide, the prosthesis will act as a constrained prosthesis, i.e. it will have only one degree of freedom in each of the anatomical planes, and the instantaneous axis of rotation will therefore be determined by the design of the prosthesis. However, if the said dimensions do not coincide, there will be a clearance "h" (Fig. 7b) which will allow more than one degree of freedom in each anatomical plane. When the said clearance "h" is within a very narrow range (no greater than 1.5 mm), we would be dealing with the case of a semi-constrained prosthesis in which the relative movement will not be determined by the design of the prosthesis so that it will be the joint action of ligaments, facet joints and muscles that determines the location of the axis of rotation. If the said clearance "h" is between 1.5 and 3 mm, this allows greater displacement without exceeding the physiological limits, and we will be faced with the third possibility of the unconstrained prosthesis.

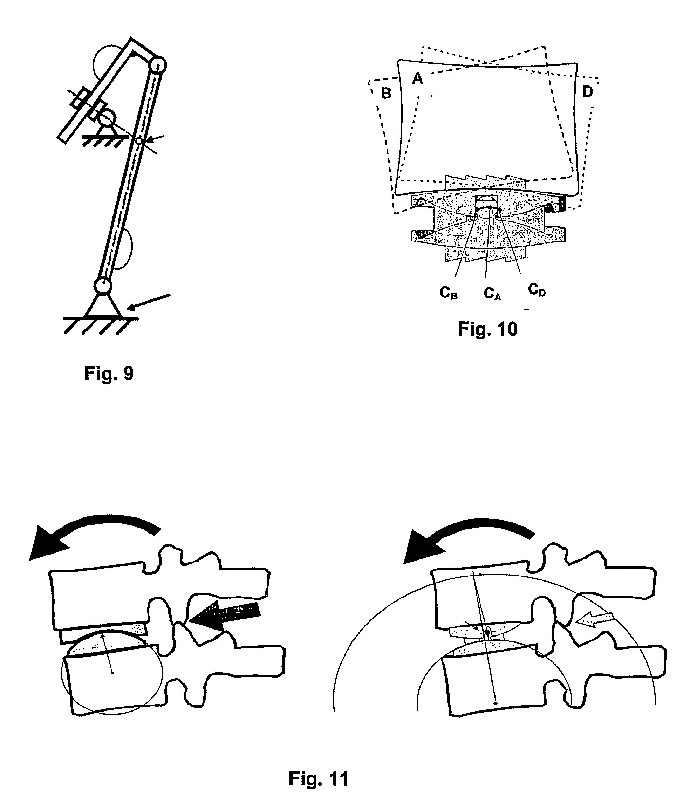

When the constrained configuration of the prosthesis is used (h = 0), the relative movement is produced both in a sagittal plane and in a frontal plane, between which the two vertebrae connected by the same may be simulated by means of the mechanism shown in Fig. 9, where one link represents the intermediate element and the other link the element anchored to the superior vertebra. If the inferior vertebra is considered fixed, the movement of the superior vertebra in each of the anatomical planes mentioned consists of one rotation plus one translation. At any moment this movement may be defined as an instantaneous rotation relative to an axis: the instantaneous axis of rotation. The position of this axis in the present prosthesis is different for each position adopted by the superior vertebra. Fig. 10 shows the successive positions adopted by the axis of rotation (C_{A}, C_{B}, C_{D}) of the flexo-extension movements performed in the plane of the figure; these positions have been represented by dots because for the said movements the axes are perpendicular to the plane of the figure. As can be proved, this axis is always located in the central zone of the intervertebral space, being displaced in one direction (anterior) when the segment flexes, and in the opposite direction (posterior) when it is extended.

This characteristic of the movement is similar to that observed by White and Panjabi in a lumbar movement unit, thus it may be considered that the present prosthesis, in its constrained configuration, enables a movement to be produced that is more similar to the physiological movement than any other of the currently existing constrained prostheses. Moreover, the location of the axis of rotation in the central zone of the intervertebral space enables the relative transversal movement between the facet joints (Fig. 11) to be minimised, with the consequent reduction in the mechanical stresses supported by the same.

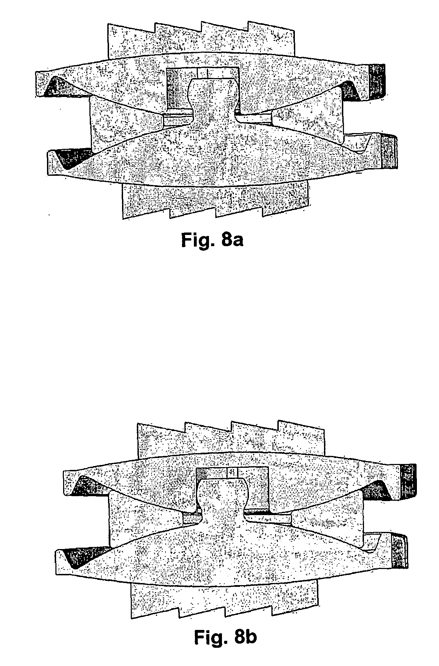

When the unconstrained configuration of the prosthesis (h≠0) is used, the relative movement between the vertebrae will have six degrees of freedom. Thus the movements of rotation permitted by the prosthesis will be independent of the translation movements. In this configuration the instantaneous axis of rotation for the movements in the different anatomical planes will not therefore be defined by the relative position between the vertebrae. Instead, as is the case in any unconstrained prosthesis design, it will be defined by the actuation of the different structures which connect both vertebrae. One problem associated with this type of design is the possible existence of excessive transversal displacements. In the present prosthesis these displacements will always be limited by the value assigned to the clearance h (Figs. 8a and 8b). In this figure the maximum anterior and posterior displacements permitted by the prosthesis may be considered. Moreover, the value of the clearance 'h' may be dimensioned independently for the antero-posterior direction and for the lateral direction (perpendicular to the antero-posterior direction), which means, for example that the hole (8) must be elliptical in shape. The maximum displacements in both directions may therefore be limited independently. Depending on the amplitude of the clearance "h", we will have a totally unconstrained prosthesis, or a semi-constrained prosthesis, according to whether the "h" has the maximum physiologically permitted amplitude or the minimum amplitude required.

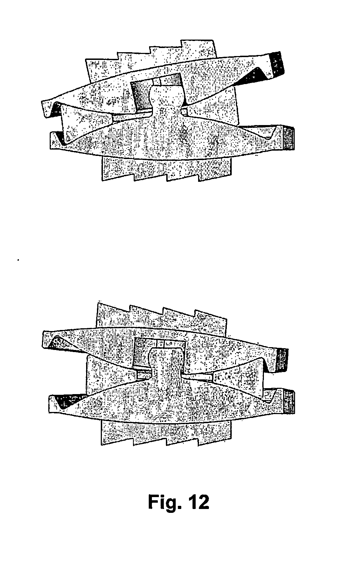

This limitation of transversal displacements will also be produced when the vertebrae connected by the prosthesis rotate. As a example, the arrangement of the different components of the prosthesis can be seen in Fig. 12, in its semi-constrained configuration, when it is in the maximum flexion and extension positions. The lateral displacement of the intermediate part (3) relative to the upper and lower parts, can also be seen.

Finally, the advantages of the advantages of the disc prosthesis of the invention are wholly conclusive and eliminate the disadvantages of the exclusively visco-elastic prostheses, and particularly the constrained prosthesis which are continuously forcing the small joints since they do not have a variable axis of positional rotation. The disadvantages of the unconstrained prostheses, such as Charité and MobyDisc, i.e. including that of permitting an exaggerated and uncontrolled horizontal displacement, are also obvious. This can be controlled with the prosthesis of the invention.

As has been previously indicated, the instantaneous axis of rotation in the relative movement between the two vertebrae of a vertebral unit or segment is continuously changing position whilst producing a flexo-extension or lateralisation movement. This continuous movement of the axis of rotation establishes a horizontal or transversal displacement of the vertebral body itself; which means that the prosthesis must be designed to be provided with these two possibilities. Consequently, that displacement is never higher than the normal physiological displacement and a device therefore exists that limits the implant in this direction.

To achieve these two aspects, the prosthesis of the invention may be unconstrained, constrained or semi-constrained, maintaining its instantaneous axis of the invention within its own intervertebral space, and which enables that feared displacement, which will always affect the articular mechanics of the posterior facets, to be limited to a certain extent. Based on personal experience of constrained prosthesis we have now been able to observe one or two cases of lumbar facetary pain indicating the tension to which the said posterior joints are subjected with this type of prosthesis.

In the prosthesis of the invention the existence of the central pivot (6), terminating in a small sphere, is what actually causes the instantaneous axis of rotation to be included within the intervertebral space, but on the other hand the clearance "h" due to the diameter of the opening (8) in the upper part (2) gives rise to the continuous change in the instantaneous axis of rotation whilst at the same allowing a limited transversal displacement. This is because, when the maximum degree of flexion or extension (approximately 12°) is reached, the intermediate part (3), which is the biconcave disc with a central opening (7) having a diameter greater than that of the central pivot (6), is displaced in one or other direction whilst serving as a maximum for limiting not only the transversal displacement but also the flexion or extension.

Depending on the diameter of the hole (8) in the upper part (2) we may therefore obtain three different configurations:
- when h=0, a constrained configuration is obtained;
- if 0 < h < 1.5 mm, a semi-constrained configuration is obtained; and
- if 1.5 < h < 3 mm, an unconstrained configuration is obtained.

### 6.3 - Wear and Strength of the Material.

Obviously all the elements constituting this implant must be sufficiently strong to be able to support all the biomechanical stresses occurring during the life of the subject wearing it. Therefore the use of metal alloys with high wear resistance and with perfect biocompatibility is therefore required as an essential element of the same. Today there do not appear to be any major problems in this sense with the chromium-cobalt-molybdenum alloys, which have been selected for this prosthesis of the invention, which have more than proved themselves in hip and knee prosthesis.

However, the same is not true for the intermediate part (3), which must be of a special material. The intermediate part (3) must be of a material which has a high degree of resistance and some good tribological characteristics in respect to the external parts. A polyethylene has therefore been selected with a special reinforcement system which is three or four times as resistant as Ultra High Molecular Weight Polyethylene. This is the so-called Cross-link Ultra High Molecular Weight Polyethylene (CLUHMWPE). This material enables intermediate parts to be obtained in which excellent polishing can be achieved to ensure suitable sliding behaviour with the two convex hemispheres (4, 5) of the parts (1, 2) due to their very low coefficient of friction, whilst logically the survival of the implant and its resistance to wear are also improved.

On the other hand, the fact that the prosthesis has two sliding surfaces (4, 5) is important in that two sliding surfaces provide better mobility and less wear than merely a single surface because the friction happens to be much lower.

Another very important aspect is that the said intermediate part (3) is rotated by a metal ring or band (13) of titanium. This band confers upon the said intermediate part a substantially greater mechanical strength and enables it to be visualised by X-ray.

This prosthesis therefore incorporates a further third articulation between the lower (1) and upper parts (2) level with the sphere (6) of the central pivot and the hole (8) of the upper part (2). This articulation therefore serves more as a pulley, giving stability to the prosthesis, than as an element providing a third sliding surface, and to a certain extent this stability may prevent the incidence of dislocations of the upper element of the same, as has in fact already occurred with the Pro-Disc prosthesis to one of the authors and to other surgeons.

### 6.4. - Paths of approach

All currently marketed prostheses are designed to be able to be implanted by an anterior approach only. Nevertheless, there are a large number of patients who, for anatomical reasons (complex situation of abdominal vessels), or because they have previously undergone abdominal operations, are excluded as candidates for a disc prosthesis. We consider it important to develop a disc prosthesis which can be implanted laterally, particularly in the space L-4 L-5, which is the most complex because of its general coincidence with the bifurcation of the iliac veins and the difficultly in displacing the latter. This prosthesis of the invention meets this requirement and, according to the orientation of the outer crests (11, 12), which incorporate both parts (1) and (2), this prosthesis can be implanted laterally or anteriorly.

### 6.5. - Selection of the type of implant.

This point relates fundamentally to the most important advantage of the prosthesis of the invention, and hence to the possibility of success of the implant. Each patient will require either a semi-constrained, constrained or unconstrained prosthesis, according to the type of degenerative discopathy suffered, his/her age, general condition of musculature and ligaments, and the degree of instability of the segment to be treated.

If an unconstrained disc prosthesis is used for a hypomobile degenerative discopathy in a young man with good musculature, the mobility of the segment will be assured, but stability may also be guaranteed due to the strong musculature, which will control the translation and rotation movements at all times.

Conversely, if we use an unconstrained disc prosthesis in a hypermobile degenerative discopathy in a young multiparous woman with poor musculature and considerable tegumentary dislocation, we will find that the vertebral segment will enjoy all the degrees of freedom for each anatomical plane, thus ensuring good mobility of the sector with continuous variation of the instantaneous axis of rotation at each stage of the movement, leaving the segment dependent on the state of elastic tension and on an intact muscular and capsule-ligamentary system, which it is assumed is defective. The stability of the segment will therefore be highly precarious and hence its effect will be to exert a negative biomechanical overload on the small joints. In this case it will be necessary to use a semi-constrained or constrained prosthesis.

Another disadvantageous situation will arise in the case of hypomobile degenerative discopathies where, if a constrained prosthesis is applied, we obtain very good stability but practically zero mobility, which is actually of no use whatsoever because we would be faced with a case similar to an intersomatic arthrodesis, which would in time produce a situation of degenerative discopathy of the suprajacent disc or Transition Syndrome.

It is therefore necessary to apply one particular type of prosthesis in each patient and use a constrained, unconstrained or semi-constrained model, according to the mechanical conditions and anatomical state of conservation of the small joints in each segment, and according to the degree of greater or lesser elasticity of the musculoligamentary system, subject to a study with dynamic X-rays, CAT or computerised axial tomography and magnetic resonance of each patient.

### 7. INDICATIONS AND CONTRAINDICATIONS OF THE PROSTHESIS

The replacement of the degenerated disc with a disc prosthesis, apart from preserving the function of the altered segment, is also intended to protect the adjacent discs from functional overload resulting from the fixing of a vertebral movement segment. Classically the indications are established for the same situations where fusion was indicated, but with a large number of exceptions or reservations, namely:
- The bone would have to be of good quality, i.e. there would have to be no osteoporosis or other form of deficit in the receptor bone.
- The discal space would have to maintain a minimum height of 5 mm so that the implant could be admitted without major tension.
- Almost all situations secondary to spondilitis or spondiolodiscitis (infections) have been rejected.
- Nor would there have to be severe intervertebral deformity or displacement.
- There would have to be no associated discal hernias.
- The path of approach (anterior) would not have to have undergone previous interventions.
- There would have to be no serious general diseases or blood discrasias.
- The patient would have to be middle-aged, between 40 and 60 approximately.

The indications have now been considerably extended, particularly in respect to the local situation and the number of segments to be treated. Degenerative discopathy and polysegmentary post-surgical instability are now accepted as indications. Nor does the association of discal hernia constitute an absolute contraindication. The same applies to degenerative scoliotic deformity, lysis or moderate lysthesis, with the exception of space L5-S1.

Summarising we can state that currently the prosthesis is indicated in the treatment of the following pathological situations of the disc:
Absolute indications:
   - Isolated monosegmentary lumbar or cervical degenerative discopathy.
   - Multisegmentary lumbar or cervical degenerative discopathy
   - Adjacent disc syndrome secondary to vertebral fusion.
Relative indications:
   - Degenerative discopathy associated with multisegmentary deformity
   - Post-fracture syndromes with discal destruction and good osseous reserve.
   - Degenerative discopathies and instabilities associated with stenosis of the channel.
   - Uni- or multilocal discogenic post-surgical instabilities
   - Post-discectomy syndromes
   - Discopathies secondary to spondilodiscitis with discal space and osseous reserve preserved, with a sufficient clinical and analytical negativisation interval of the infection.
   - Discopathy associated with non-extruded discal hernia.
   - Situations of absolute indication associated with obesity, smoking, diabetes or general or local illness involving increased risk.

However, contraindications of a general nature, serious or tumorous disease, blood discrasias, pregnancy, etc., difficulties relating to the path of approach, previous operation for serious abdominal problems, etc., or unsuitable age of the patient, above or below, remain absolute contradictions.

### 8. TECHNIQUE OF IMPLANTING THE PROSTHESIS

The actual technique commences with the preparation of the disc, cleaning and removal of the cartilaginous plate from the vertebral plates until the fibrous discal ring is reached throughout its lateral and posterior perimeter.

Once the disc is prepared the distraction pins or rods are inserted at the predetermined points with a centring instrument and the space is distracted with the aid of the distractor forceps and the expanding clips so as not to harm the vertebral body.

The height, width and weight of the prosthesis are then determined with the testers and the grooves of the prosthesis anchorage crests are measured with the double tuning fork scopes.

Maintaining the final height of the implant with the distractor, the prosthesis "packed" in the introducing carrier clip is then inserted. The final stage would be the radioscopic verification of the correct position of the implant and closing of the operative wound.

Now that the nature of this invention has been adequately described, together with a practical application of the same, all we need add is that both its shape and the materials and execution of the same are subject to modifications provided that they do not substantially affect that characteristics claimed in the following.

## Claims

1. A universally applicable intervertebral disc prosthesis of the type formed by three parts:
- a lower part (1) being lenticular in shape in its vertical section, and kidney-shaped in a plan view, with an upper surface (4) in the form of a spherical cap which terminates in a circular retaining edge (14), and with a lower surface (9), also in the shape of a spherical cap with a curvature
wherein the radius of the upper surface (4) is smaller than that of the lower surface (9), and terminating in a flat edge;
- an upper part (2) being lenticular in shape in its vertical section, and kidney-shaped in a plan view, with a lower surface (5) in the form of a spherical cap which terminates in a circular retaining edge (14), and with an upper surface (10), also in the form of a spherical cap with a curvature
wherein the radius of the lower surface (5) is smaller than that of the upper surface (10), and terminating in a flat edge; and
- a biconcave toroidal intermediate part (3) which articulates on the other two lower (1) and upper (2) parts by means of upper and lower cavities of this intermediate part (3), which are spherical with substantially the same curvature as the said upper and lower surfaces (4, 5) of the lower (1) and upper (2) parts respectively, to ensure that they are in contact throughout their area when the said parts (1, 2, 3) are mounted in their operative position;
wherein,
- above the spherical surface (4) of the lower part (1) there is a pivot (6) with a radially orientated spherical head;
- the upper part (2) has a hole (8) that is circular or elliptical in shape;
- the said spherical head of the said pivot (6) and the said_hole_(8) forming a third articulation;
- **characterised in that,**
- the intermediate part (3) has a central opening (7) that is circular in shape with dimensions larger than those of the hole (8) in the said upper part (2);
- when the three parts (1, 2, 3) that comprise the prosthesis are mounted in their operative position, the said pivot, with the spherical head (6), crosses the intermediate part (3) via its central opening (7), and is accommodated in the hole (8) made in the upper part (2), the said spherical head of the said pivot (6) and the said hole (8) forming a pulley which provides the disc prosthesis with stability.

2. The intervertebral disc prosthesis according to Claim 1, **characterised in that** between the said spherical head of the said pivot (6) and the said hole (8) there is a type of clearance (h) as follows:
- without clearance (h), where the diameter of the spherical head of the said pivot (6) is substantially equal to the diameter of the hole (8), which means that the prosthesis is a constrained prosthesis which only permits one degree of freedom in each anatomical plane, and where the position of the axis of rotation of one vertebra of a vertebral segment relative to another vertebra of the said vertebral segment is determined by the design of the prosthesis;
- with limited clearance (h) in the approximate range of 0 to 1.5 mm, which means that the prosthesis is a semi-constrained prosthesis which permits more than one degree of freedom in each anatomical plane, which permits a slight transversal displacement of one vertebra relative to the other vertebra of the same vertebral segment, and where the position of the said axis of rotation is determined not only by the prosthesis but also by the joint action of ligaments, facet joints and muscles; and
- with limited clearance (h) in the approximate range of 1.5 to 3 mm, which means that the prosthesis is an unconstrained prosthesis which permits more than one degree of freedom in each anatomical plane, which permits maximum transversal displacement without exceeding the physiological limits, of one vertebral relative to the other vertebra of the same vertebral segment, and where the position of the said axis of rotation is determined not only by the prosthesis but also by the joint action of ligaments, facet joints and muscles.

3. The intervertebral disc prosthesis according to Claim 2, **characterised in that** the said clearance (h) may be dimensioned with one value for the antero-posterior direction of the vertebral segment and another value for the lateral direction of the vertebral segment.

4. The intervertebral disc prosthesis according to the previous claims, **characterised in that** the said pivot with the spherical head (6) and the hole (8) are displaced in the sagittal plane relative to a centered position so that the range of mobility of the prosthesis in flexion is greater than in extension.

5. The intervertebral disc prosthesis according to Claim 1, **characterised in that** the said lower (1) and upper (2) parts are formed by a biocompatible metal alloy with a high wear resistance, specifically of chromium-cobalt-molybdenum, and
wherein the said lower surfaces (9) of the said lower part (1) and upper surface surfaces (10) of the said upper part (2) are covered by a biocompatible, easily osteo-integrated material, specifically a porous tantalum plasma.

6. The intervertebral disc prosthesis according to Claim 1, **characterised in that** the said intermediate part (3) is formed by a material with a high degree of resistance and good tribological characteristics in respect to the lower and upper parts (1, 2), consisting of "cross link" ultra high molecular weight polyethylene (CLUHMWPE), and **in that** the said intermediate part (3) is rotated by a metal ring or band (13) of titanium which gives it greater resistance and enables it to be visualised by X-ray.

7. The intervertebral disc prosthesis according to Claim 1, **characterised in that** both of the said surfaces (9) of the lower part (1) and (10) of the upper part (2) are provided in the vertical direction with a pair of crests or spikes (11) and (12) respectively, which are parallel to each other, with two possible orientations according to the path of approach selected:- antero-posterior for its implantation anteriorly; and - lateral for its implantation laterally.

8. The intervertebral disc prosthesis according to Claim 1, **characterised in that** the said circular retaining edges (14) limit the displacement of the said intermediate part (3) and hence the maximum degree of flexion, extension and lateral flexion permitted by the said disc prosthesis.

## Patentansprüche

1. Universell anwendbare Bandscheibenprothese von der aus drei Teilen bestehenden Art:
- einem unteren Teil (1), der im Vertikalschnitt eine linsenförmige Gestalt und in der Draufsicht eine nierenförmige Gestalt aufweist, mit einer Oberseite (4) in Form einer kugelförmigen Kappe, die in einer kreisförmigen Haltekante (14) endet, und mit einer Unterseite (9), die ebenfalls die Form einer kugelförmigen Kappe mit einer Krümmung aufweist, wobei der Radius der Oberseite (4) kleiner ist als der Radius der Unterseite (9), und in einer flachen Kante endet;
- einem oberen Teil (2), der im Vertikalschnitt eine linsenförmige Gestalt und in der Draufsicht eine nierenförmige Gestalt aufweist, mit einer Unterseite (5) in Form einer kugelförmigen Kappe, die in einer kreisförmigen Haltekante (14) endet, und mit einer Oberseite (10), die ebenfalls die Form einer kugelförmigen Kappe mit einer Krümmung aufweist, wobei der Radius der Unterseite (5) kleiner ist als der Radius der_Oberseite (10), und in einer flachen Kante endet; und
- einem bikonkaven ringförmigen Zwischenteil (3), der gelenkig auf den anderen beiden Teilen, dem unteren Teil (1) und dem oberen Teil (2) angeordnet ist, durch obere und untere Hohlräume dieses Zwischenteils (3), die kugelförmig sind und im Wesentlichen dieselbe Krümmung wie die Ober- und Unterseiten (4, 5) des oberen (1) bzw. unteren Teils (2) aufweisen, um sicherzustellen, dass sie über ihre gesamte Fläche in Berührung sind, wenn die Teile (1, 2, 3) in ihrer Betriebsstellung angebracht werden;
wobei
- sich oberhalb der kugelförmigen Fläche (4) des unteren Teils (1) ein Drehzapfen (6) mit einem radial orientierten Kugelkopf befindet;
- der obere Teil (2) ein Loch (8) mit einer kreisförmigen oder elliptischen Gestalt aufweist;
- der Kugelkopf des Drehzapfens (6) und das Loch (8) eine dritte Gelenkverbindung bilden;
**dadurch gekennzeichnet, dass**
- der Zwischenteil (3) eine zentrale Öffnung (7) aufweist, die von kreisförmiger Gestalt ist und Abmessungen hat, die größer sind als die Abmessungen des Loches (8) im oberen Teil (2);
- der Drehzapfen mit dem Kugelkopf (6) den Zwischenteil (3) über seine mittlere Öffnung (7) kreuzt, wenn die drei Teile (1, 2, 3), aus denen die Prothese, besteht, in ihrer Betriebsstellung montiert werden, und im Loch (8) im oberen Teil (2) untergebracht ist, wobei der Kugelkopf des Drehzapfens (6) und das Loch (8) eine Rolle formen, die der Bandscheibenprothese Stabilität verleiht.

2. Bandscheibenprothese nach Anspruch 1, **dadurch gekennzeichnet, dass** zwischen dem Kugelkopf des Drehzapfens (6) und dem Loch (8) eine Art von Spiel (h) wie folgt besteht:
- ohne Spiel (h), wobei der Durchmesser des Kugelkopfes des Drehzapfens (6) im Wesentlichen dem Durchmesser des Loches (8) entspricht, was bedeutet, dass die Prothese eine eingezwängte Prothese ist, die nur einen Freiheitsgrad in jeder anatomischen Ebene gestattet, und wobei die Position der Drehachse eines wirbels eines Wirbelsegments relativ zu einem anderen Wirbel des Wirbelsegments vom Design der Prothese bestimmt wird;
- mit begrenztem Spiel (h) im ungefähren Bereich von 0 bis 1,5 mm, was bedeutet, dass die Prothese eine halb eingezwängte Prothese ist, die mehr als einen Freiheitsgrad in jeder anatomischen Ebene gestattet, wodurch eine leichte Querverschiebung eines Wirbels relativ zum anderen Wirbel desselben Wirbelsegments möglich ist, und wobei die Position der Drehachse nicht nur von der Prothese, sondern auch von der gemeinsamen Wirkung von Bändern, Facettengelenken und Muskeln bestimmt wird; und
- mit begrenztem Spiel (h) im ungefähren Bereich von 1,5 bis 3 mm, was bedeutet, dass die Prothese eine uneingezwängte Prothese ist, die mehr als einen Freiheitsgrad in jeder anatomischen Ebene gestattet, wodurch eine maximale Querverschiebung ohne Überschreitung der physiologischen Grenzen eines Wirbels relativ zum anderen Wirbel desselben Wirbelsegments möglich ist, und wobei die Position der Drehachse nicht nur von der Prothese, sondern auch von der gemeinsamen Wirkung von Bändern, Facettengelenken und Muskeln bestimmt wird.

3. Bandscheibenprothese nach Anspruch 2, **dadurch gekennzeichnet, dass** das Spiel (h) mit einem Wert für die anteroposteriore Richtung des Wirbelsegments und einem anderen Wert für die seitliche Richtung des Wirbelsegments dimensioniert sein kann.

4. Bandscheibenprothese nach den vorhergehenden Ansprüchen, **dadurch gekennzeichnet, dass** der Drehzapfen mit dem Kugelkopf (6) und dem Loch (8) in der sagittalen Ebene relativ zu einer zentrierten Stellung verschoben ist, so dass der Mobilitätsbereich der Prothese in Beugung größer ist als der in Streckung.

5. Bandscheibenprothese nach Anspruch 1, **dadurch gekennzeichnet, dass** der untere (1) und der obere (2) Teil aus einer biokompatiblen Metalllegierung mit hoher Abriebfestigkeit, insbesondere aus Chrom-Kobalt-Molybdän bestehen, und wobei die Unterseiten (9) des unteren Teils (1) und die Oberseite (10) des oberen Teils (2) von einem biokompatiblen leicht osteointegrierten Material, insbesondere porösem Tantalplasma, bedeckt sind.

6. Bandscheibenprothese nach Anspruch 1, **dadurch gekennzeichnet, dass** der Zwischenteil (3) aus einem Material mit hohem Widerstandsgrad und guten tribologischen Eigenschaften bezüglich des unteren und oberen Teils (1, 2) besteht, das aus "vernetztem" ultrahochmolekularem Polymethylen (CLUHMWPE) besteht, und dass der Zwischenteil (3) von einem Metallring oder Metallband (13) aus Titan gedreht wird, der bzw. das ihm größeren Widerstand verleiht und ihn unter Röntgenstrahlen sichtbar macht.

7. Bandscheibenprothese nach Anspruch 1, **dadurch gekennzeichnet, dass** sowohl die Seite (9) des unteren Teils (1) als auch die Seite (10) des oberen Teils (2) in vertikaler Richtung mit einem Paar Kronen oder Dornen (11) bzw. (12) versehen sind, die parallel zueinander liegen, mit zwei möglichen Orientierungen je nach gewähltem Zugangspfad: anteroposterior für die anteriore Implantation und lateral für die laterale Implantation.

8. Bandscheibenprothese nach Anspruch 1, **dadurch gekennzeichnet, dass** die kreisförmigen Haltekanten (14) die Verschiebung des Zwischenteils (3) und somit den maximalen Grad der Biegung, Streckung und seitlichen Biegung, die die Bandscheibenprothese zulässt, begrenzen.

## Revendications

1. Prothèse de disque intervertébral à application universelle du type formé de trois parties :
- une partie inférieure (1) qui a une forme lenticulaire dans sa section verticale, et la forme d'un haricot vue de dessus, avec une surface supérieure (4) se présentant sous la forme d'un chapeau sphérique qui se termine par un bord de retenue circulaire (14), et avec une surface inférieure (9), se présentant aussi sous la forme d'un chapeau sphérique possédant une courbure, le rayon de la surface supérieure (4) étant plus petit que celui de la surface inférieure (9), et se terminant par un bord plat ;
- une partie supérieure (2) qui a une forme lenticulaire dans sa section verticale, et la forme d'un haricot vue de dessus, avec une surface inférieure (5) se présentant sous la forme d'un chapeau sphérique qui se termine par un bord de retenue circulaire (14), et avec une surface supérieure (10), se présentant aussi sous la forme d'un chapeau sphérique possédant une courbure, le rayon de la surface inférieure (5) étant plus petit que celui de la surface supérieure (10), et se terminant par un bord plat ; et
- une partie intermédiaire toroïdale biconcave (3) qui s'articule sur les deux autres parties inférieure (1) et supérieure (2) au moyen des cavités supérieure et inférieure de cette partie intermédiaire (3), qui sont sphériques avec sensiblement la même courbure que lesdites surfaces supérieure et inférieure (4, 5) des parties respectivement inférieure (1) et supérieure (2), pour s'assurer qu'elles sont en contact sur toute leur surface quand lesdites parties (1, 2, 3) sont montées dans leur position de fonctionnement ;
dans laquelle
- au-dessus de la surface sphérique (4) de la partie inférieure (1) se trouve un pivot (6) possédant une tête sphérique orientée radialement ;
- la partie supérieure (2) présente un trou (8) dont la forme est circulaire ou elliptique ;
- ladite tête sphérique dudit pivot (6) et ledit trou (8) formant une troisième articulation ;
- **caractérisée en ce que,**
- la partie intermédiaire (3) a une ouverture centrale (7) dont la forme est circulaire avec des dimensions plus grandes que celles du trou (8) dans ladite partie supérieure (2) ;
- quand les trois parties (1, 2, 3) qui constituent la prothèse sont montées dans leur position de fonctionnement, ledit pivot, avec la tête sphérique (6), croise la partie intermédiaire (3) par le biais de son ouverture centrale (7), et est logé dans le trou (8) réalisé dans la partie supérieure (2), ladite tête sphérique dudit pivot (6) et ledit trou (8) formant une poulie qui fournit à la prothèse de disque une stabilité.

2. Prothèse de disque intervertébral selon la revendication 1, **caractérisée en ce qu'**entre ladite tête sphérique dudit pivot (6) et ledit trou (B) il y a un certain type de jeu (h), comme suit :
- sans jeu (h), lorsque le diamètre de la tête sphérique dudit pivot (6) est sensiblement égal au diamètre du trou (8), ce qui signifie que la prothèse est une prothèse contrainte qui ne permet qu'un degré de liberté dans chaque plan anatomique, et lorsque la position de l'axe de rotation d'une vertèbre d'un segment vertébral par rapport à à une autre vertèbre dudit segment vertébral est déterminée par la conception de la prothèse ;
- avec un jeu limité (h) d'environ 0 à 1,5 mm, ce qui signifie que la prothèse est une prothèse semi-contrainte qui permet plusieurs degré de liberté dans chaque plan anatomique, autorisant un léger déplacement transversal d'une vertèbre par rapport à l'autre vertèbre du même segment vertébral, et lorsque la position dudit axe de rotation est déterminée non seulement par la prothèse mais aussi par l'action conjuguée des ligaments, des articulations à facettes et des muscles ; et
- avec un jeu limité (h) d'environ 1,5 à 3 mm, ce qui signifie que la prothèse est une prothèse non-contrainte qui permet plusieurs degrés de liberté dans chaque plan anatomique, autorisant un déplacement transversal maximal sans dépasser les limites physiologiques, d'une vertèbre par rapport à l'autre vertèbre du même segment vertébral, et lorsque la position dudit axe de rotation est déterminée non seulement par la prothèse mais aussi par l'action conjuguée des ligaments, des articulations à facettes et des muscles.

3. Prothèse de disque intervertébral selon la revendication 2, **caractérisée en ce que** ledit jeu (h) peut être dimensionné avec une valeur pour la direction antéro-postérieure du segment vertébral et une autre valeur pour la direction latérale du segment vertébral.

4. Prothèse de disque intervertébral selon les revendications précédentes, **caractérisée en ce que** ledit pivot possédant la tête sphérique (6) et le trou (8) sont déplacés dans le plan sagittal par rapport à une position centrée de sorte que l'étendue de la mobilité de la prothèse en flexion est supérieure à celle en extension.

5. Prothèse de disque intervertébral selon la revendication 1, **caractérisée en ce que** lesdites parties inférieure (1) et supérieure (2) sont réalisées dans un alliage en métal biocompatible présentant une résistance élevée à l'usure, spécifiquement en chrome-cobalt-molybdène, et dans laquelle lesdites surfaces inférieures (9) de la ladite partie inférieure (1) et les surfaces de la surface supérieure (10) de ladite partie supérieure (2) sont recouvertes d'un matériau biocompatible facilement ostéointégré, spécifiquement un plasma de tantale poreux.

6. Prothèse de disque intervertébral selon la revendication 1, **caractérisée en ce que** ladite partie intermédiaire (3) est réalisée dans un matériau présentant un degré élevé de résistance et de bonnes caractéristiques tribologiques en ce qui concerne les parties inférieure et supérieure (1, 2), comportant du polyéthylène « réticulé » de poids moléculaire ultraélevé (PE-HMG), et **en ce que** ladite partie intermédiaire (3) est tournée par une bague en métal ou bande (13) de titane qui lui confère une plus grande résistance et lui permet d'être visualisée aux rayons X.

7. Prothèse de disque intervertébral selon la revendication 1, **caractérisée en ce que** à la fois lesdites surfaces (9) de la partie inférieure (1) et (10) de la partie supérieure (2) sont munies, dans la direction verticale, d'une paire respective de crêtes ou pointes (11) et (12), qui sont parallèles l'une par rapport à l'autre, avec deux orientations possibles selon l'abord sélectionné : - antéro-postérieur pour son implantation antérieure; et - latéral pour son implantation latérale.

8. Prothèse de disque intervertébral selon la revendication 1, **caractérisée en ce que** lesdits bords de retenue circulaires (14) limitent le déplacement de ladite partie intermédiaire (3) et par conséquent le degré maximal de flexion, d'extension et de flexion latérale permis par ladite prothèse de disque.
